(19) Europäisches Patentamt
European Patent Office
Office européen des brevets

(11) Veröffentlichungsnummer : **0 062 844**
**B1**

(12) **EUROPÄISCHE PATENTSCHRIFT**

(45) Veröffentlichungstag der Patentschrift : **28.11.84**

(21) Anmeldenummer : **82102756.2**

(22) Anmeldetag : **01.04.82**

(51) Int. Cl.³ : **C 07 D221/14**, C 07 D401/12, C 07 D413/12, C 07 D417/12, A 61 K 31/435, A 61 K 31/495

(54) **Substituierte Benzo(de)isochinoline, Verfahren zu ihrer Herstellung und ihre Verwendung als Arzneimittel.**

(30) Priorität : **09.04.81 DE 3114400**
**27.05.81 DE 3121082**

(43) Veröffentlichungstag der Anmeldung : **20.10.82 Patentblatt 82/42**

(45) Bekanntmachung des Hinweises auf die Patenterteilung : **28.11.84 Patentblatt 84/48**

(84) Benannte Vertragsstaaten : **AT BE CH DE FR GB IT LI NL SE**

(56) Entgegenhaltungen :
**FR-A- 1 549 630**
**US-A- 3 291 829**
**CHEMICAL ABSTRACTS, Band 96, Nr. 7, 15. Februar 1982, Seite 606, Nr. 52154e, Columbus, Ohio, USA, N. VISWANATHAN et al.: "Synthesis and hypotensive activity of some aminoguanidines"**
**Die Akte enthält technische Angaben, die nach dem Eingang der Anmeldung eingereicht wurden und die nicht in dieser Patentschrift enthalten sind.**

(73) Patentinhaber : **Beiersdorf Aktiengesellschaft**
**Unnastrasse 48**
**D-2000 Hamburg 20 (DE)**

(72) Erfinder : **Cohnen, Erich, Dr. Dipl.-Chem.**
**Hellwigstrasse 25**
**D-2000 Hamburg 20 (DE)**
Erfinder : **Armah, Ben, Dr.**
**Milcher Strasse 9d**
**D-2000 Hamburg 52 (DE)**

**EP 0 062 844 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue substituierte Benzo[de]isochinoline der allgemeinen Formel I

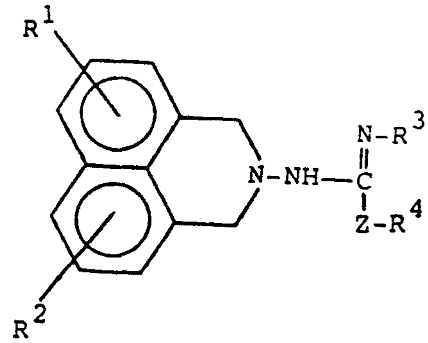

(I)

in der

$R^1$ und $R^2$, die gleich oder verschieden sein können, ein Wasserstoff- oder Halogenatom oder eine Alkoxygruppe mit 1 bis 4 Kohlenstoffatomen oder

$R^1$ und $R^2$ zusammen die Äthylengruppe,

Z Sauerstoff, Schwefel, die Methylengruppe oder die Gruppe N—$R^5$, und entweder

$R^3$ und $R^4$ zusammen die Gruppe $—(CH_2)_n—$ bedeuten, worin n die Zahl 2 oder 3 sein kann und wobei $R^5$ Wasserstoff ist (Verbindungen A),

oder, wenn Z die Gruppe N—$R^5$ ist, $R^3$, $R^4$ und $R^5$, die gleich oder verschieden sein können, ein Wasserstoffatom oder eine Alkylgruppe mit 1 bis 4 Kohlenstoffatomen bedeuten (Verbindungen B) sowie deren Säureadditionssalze, Verfahren zu ihrer Herstellung und ihre Verwendung als Arzneimittel.

Verbindungen (A) erhält man mit der Definition, daß die Reste $R^3$ und $R^4$ zusammen die Alkyliden-Gruppen $—(CH_2)_n—$ bedeuten, worin n die Zahl 2 oder 3 ist. Z ist in diesem Fall Sauerstoff, Schwefel, die Methylengruppe oder die Iminogruppe und $R^5$ ist Wasserstoff, so daß heterocyclische Ringe unter Einbeziehung der >C=N—Gruppe, insbesondere Imidazoline, Tetrahydropyrimidine, Dihydropyrrole, Oxazoline und Thiazoline, gebildet werden.

Verbindungen (B) erhält man mit der Definition, daß Z die Gruppe N—$R^5$ bedeutet, wobei $R^5$ die oben angegebenen Bedeutungen wie auch $R^3$ oder $R^4$ hat. Es sind (Benzo[de]isochinolin-2-yl)-guanidine.

Bevorzugt werden Verbindungen (A) der allgemeinen Formel I in der $R^1$ und $R^2$ Wasserstoff oder $R^1$ und/oder $R^2$ die Methoxygruppe oder ein Chloratom bedeuten. Substituenten befinden sich vorzugsweise in 6-Stellung und wenn $R^1$ und $R^2$ zusammen eine Äthylengruppe darstellen, befindet sich diese in der 6,7-Stellung. Weiterhin ist Z vorzugsweise die Iminogruppe. Bevorzugt werden Verbindungen (A) der Formel I, in der n die Zahl 2 bedeutet.

Bevorzugt werden Verbindungen (B) der allgemeinen Formel I, in der $R^1$ und $R^2$ Wasserstoff oder $R^1$ oder $R^2$ die Methoxygruppe oder ein Halogenatom, insbesondere ein Chloratom bedeuten. Vorzugsweise befinden sich die Substituenten in der 6-Stellung. Wenn $R^1$ und $R^2$ zusammen eine Äthylengruppe darstellen, befindet sich diese vorzugsweise in 6,7-Stellung. Die Substituenten $R^3$, $R^4$ und $R^5$ sind vorzugsweise Wasserstoffatome oder Methylgruppen, wobei $R^3$ bevorzugt ein Wasserstoffatom ist.

Die erfindungsgemäßen Substanzen und ihre Salze besitzen wertvolle therapeutische Eigenschaften. Sie üben eine sympathicomimetische Wirkung aus und rufen eine langanhaltende Blutdrucksteigerung hervor. Sie können daher als Antihypotonica verwendet werden.

In der FR-A-1 549 630 sind Verbindungen beschrieben, die zwar den anmeldungsgemäßen Verbindungen strukturell sehr ähnlich sind, aber demgegenüber die gegensätzliche, nämlich antihypertensive, Wirkung aufweisen.

Die Dosierungen der Verbindungen (A) liegen bei i. a. und p. o. Applikation im Bereich von 1-10 mg/kg bei Katzen. Zur Behandlung hypotoner Zustände beim Menschen werden die Verbindungen in Dosierungen von 10-50 mg pro Tag angewendet.

Die Dosierungen der Verbindungen (B) liegen bei i. a. und p. o. Applikation im Bereich von 0,1-1 mg/kg bei Katzen. Zur Behandlung hypotonor Zustände beim Menschen werden die Substanzen in Dosierungen von 1-5 mg pro Tag angewendet.

Gemäß der Erfindung werden pharmazeutische Zusammensetzungen geschaffen, die eine Verbindung der Formel I oder deren Salze, zusammen mit einem pharmazeutisch verträglichen Verdünnungsmittel oder Träger enthalten.

Die Verbindungen gemäß der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können oral in Form von Tabletten, Dragees, Sirups, Suspensionen und Flüssigkeiten, oder parenteral in Form von Lösungen oder Suspensionen verabreicht werden. Oral zu verabreichende Präparate können ein oder mehrere Zusätze, wie Süßungsmittel,

Aromatisierungsmittel, Farbstoffe und Konservierungsmittel, enthalten. Tabletten können den Wirkstoff mit üblichen, pharmazeutisch verträglichen Hilfsmitteln vermischt enthalten, z. B. inerten Verdünnungsmitteln wie Calciumcarbonat, Natriumcarbonat, Lactose und Talk, Granulierungsmitteln und Mitteln, die den Zerfall der Tabletten bei oraler Verabreichung fördern, wie Stärke oder Alginsäure, Bindemitteln wie Stärke oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Äthanol, Propylenglycol Äther des Tetrahydrofurfurylalkohols und Wasser.

Die Tabletten können nach bekannten Arbeitsweisen überzogen werden, um den Zerfall und die Resorption im Magen-Darmtrakt zu verzögern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind. z. B. Suspendiermittel wie Methylcellulose, Tragacanth oder Natriumalginat, Netzmitteln wie Lecithin, Polyoxyäthylenstearat und Polyoxyäthylensorbitanmonooleat und Konservierungsmitteln wie Äthylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert. Die pharmazeutischen Präparate können den Wirkstoff in einer Menge von 0,1 bis 90 %, insbesondere 1 bis 90 %, enthalten, wobei der Rest ein Trägerstoff oder Zusatzstoff ist. Im Hinblick auf die Herstellung und Verabreichung werden feste Präparate, wie Tabletten und Kapseln, bevorzugt. Vorzugsweise enthalten die Präparate den Wirkstoff in einer Menge von 1-50 mg entsprechend den bevorzugten Tagesdosen.

Die neuen Verbindungen der allgemeinen Formel I können nach folgenden Verfahren hergestellt werden :

a) Verbindungen (A) der allgemeinen Formel I, worin Z für ein Sauerstoff- oder Schwefelatom steht, erhält man durch Umsetzung von Verbindungen der allgemeinen Formel II

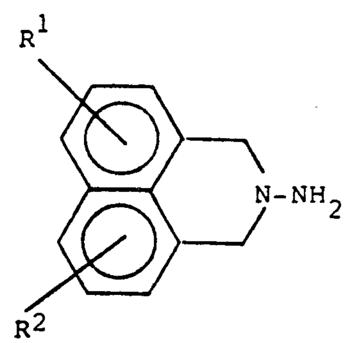

(II)

worin $R^1$ und $R^2$ die obengenannte Bedeutung haben, mit Halogenalkylisocyanaten oder Halogenalkylisothiocyanaten der allgemeinen Formel III

$$Z = C{=}N{-}(CH_2)_n{-}Y \qquad \text{(III)}$$

in der Z ein Sauerstoff- oder Schwefelatom bedeutet und in der n die oben genannte Bedeutung hat und Y ein Chlor-, Brom- oder Jodatom ist.

Dabei entstehen zunächst Verbindungen der allgemeinen Formel IV

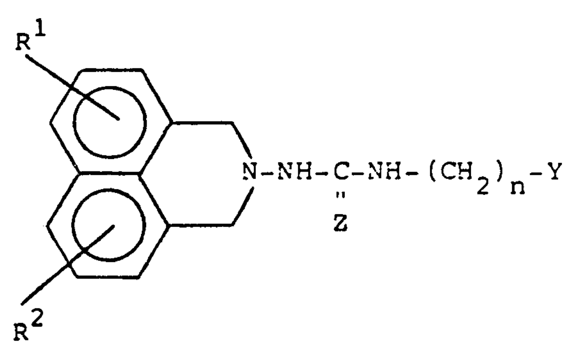

(IV)

in der $R^1$, $R^2$, Z, n und Y die angegebene Bedeutung haben, die sich zu Verbindungen der Formel I cyclisieren lassen.

Die Umsetzung der Verbindungen gemäß der Formel II mit den halogenisocyanaten bzw. Halogenisothiocyanaten erfolgt vorzugsweise bei Raumtemperatur oder leicht erhöhter Temperatur in einem Lösungsmittel, z. B. Toluol.

Die erhaltenen Harnstoffe bzw. Thioharnstoffe der Formel IV lassen sich durch Erhitzen in wäßriger oder alkoholischer Lösung cyclisieren.

b) Verbindungen (A) der Formel I, in der Z die Methylengruppe bedeutet, sind zugänglich durch Reaktion von Verbindungen der Formel II

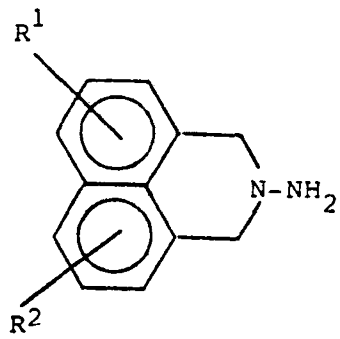

(II)

in der $R^1$ und $R^2$ die angegebene Bedeutung haben, mit Iminoäthern der allgemeinen Formel V

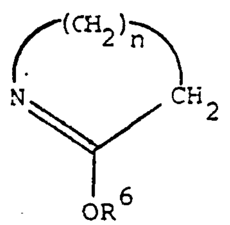

(V)

in der $R^6$ eine niedere Alkylgruppe mit vorzugsweise 1 bis 4 Kohlenstoffatomen ist und n die oben genannte Bedeutung hat.

Die Reaktion erfolgt durch Erhitzen der Reaktionspartner ohne Lösungsmittel oder unter Verwendung eines inerten Lösungsmittels wie z. B. Toluol oder Xylol in einem stöchiometrischen Verhältnis oder mit einem Überschuß des Iminoäthers.

c) Verbindungen (A) der allgemeinen Formel I, in der Z die Iminogruppe bedeutet, erhält man durch Umsetzung von Verbindungen der Formel II

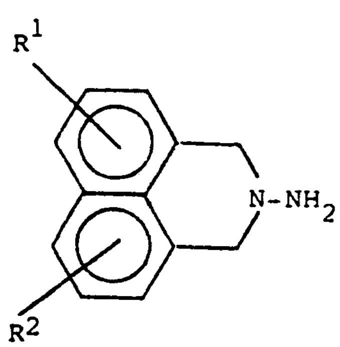

(II)

in der $R^1$ und $R^2$ die angegebene Bedeutung haben, mit Benzoylisothiocyanat zu den entsprechenden Benzoylthioharnstoffen, die durch Abspalten des Benzoylrests in die entsprechenden Thioharnstoffe überführt werden, und anschließende Alkylierung und Cyclisierung mit Alkylendiaminen.

Vorzugsweise bildet man das Benzoylisothiocyanat aus Ammoniumthiocyanat und Benzoylchlorid bei Raumtemperatur und gibt anschließend das Isochinolin der Formel II bei gleicher Temperatur zu dem Reaktionsgemisch.

Zur hydrolytischen Abspaltung der Benzoylgruppe wird der erhaltene Benzoylthioharnstoff vorzugsweise mit alkoholischer Natronlauge erwärmt.

Die erhaltenen Thioharnstoffe werden durch Alkylierung, vorzugsweise mit Methyljodid oder Dimethylsulfat, in die S-Alkylisothiuronium-Salze, vorzugsweise die S-Methylisothiuronium-Salze, der allgemeinen Formel VI

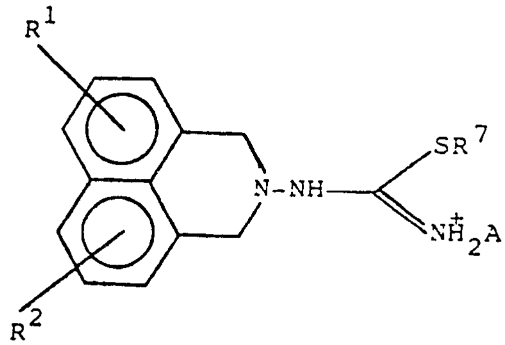

(VI)

überführt, in der $R^7$ eine niedere Alkylgruppe mit vorzugsweise 1 bis 4 Kohlenstoffatomen, A ein Anion, beispielsweise ein Jodid- oder Methylsulfat-Ion ist und $R^1$ und $R^2$ die vorstehende Bedeutung haben.

Die Reaktion wird vorzugsweise in alkoholischer Lösung bei Siedetemperatur durchgeführt.

Die erhaltenen Isothiuroniumsalze der allgemeinen Formel VI werden durch Umsetzung mit 1,2-Diaminoäthan in 2-Imidazoline und durch Umsetzung mit 1,3-Diaminopropan in Tetrahydro-Pyrimidine überführt.

Die Kondensationsreaktion wird vorzugsweise in niederen Alkoholen als Lösungsmittel bei Siedetemperatur vorgenommen.

Die neuen Verbindungen (B) der allgemeinen Formel I können nach folgenden Verfahren hergestellt werden :

d) durch Umsetzung von Verbindungen der allgemeinen Formel II

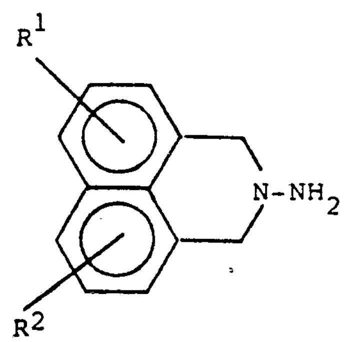

(II)

in der $R^1$ und $R^2$ die oben angegebene Bedeutung haben, mit Cyanamiden. Die Umsetzung erfolgt in Alkoholen, bevorzugt n-Amylalkohol, bei Siedetemperatur. Mit Cyanamid erhält man die mit $R^3 = R^4 = R^5 = H$ unsubstituierten Guanidine, während mit den mit $R^3$, $R^4$ und $R^5$ entsprechend substituierten Cyanamiden die Einführung der Substituenten $R^3$, $R^4$ und $R^5$ gelingt.

e) durch Reaktion von Verbindungen der allgemeinen Formel II mit der angegebenen Bedeutung von $R^1$ und $R^2$, mit aliphatischen Isothiocyanaten zu Thiosemicarbaziden der allgemeinen Formel VII

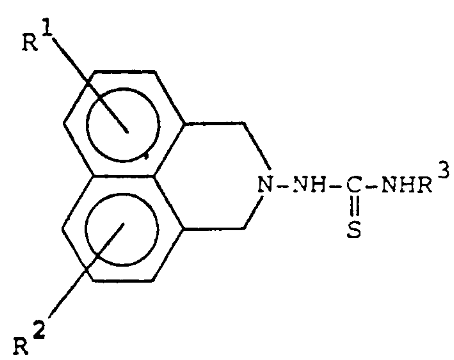

(VII)

in der $R^1$, $R^2$ und $R^3$ die angegebene Bedeutung haben, anschließende S-Methylierung mit Methyljodid und nachfolgende Umsetzung mit Ammoniak oder primären oder sekundären Aminen zur Einführung der Reste $R^4$ und $R^5$ unter Abspaltung des Thiosubstituenten.

f) durch Reaktion von Verbindungen der allgemeinen Formel II mit der dort angegebenen Bedeutung

von $R^1$ und $R^2$ mit Benzoylisothiocyanat zu Thiosemicarbaziden der allgemeinen Formel VIII

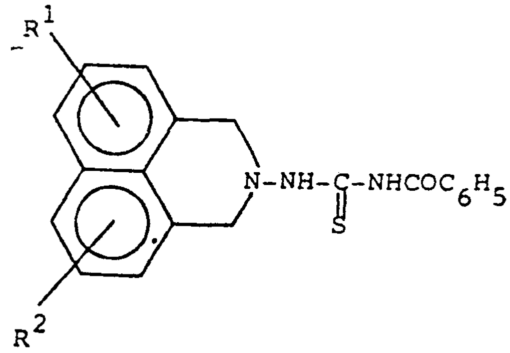

(VIII)

In einem zweiten Schritt erfolgt die Umwandlung in Verbindungen der allgemeinen Formel IX

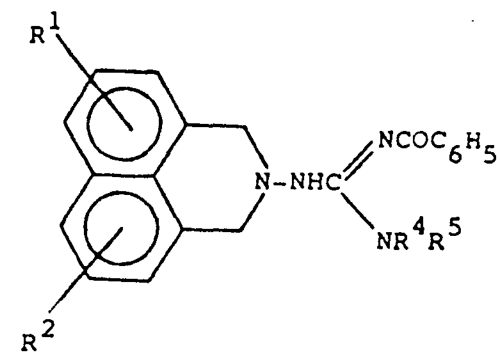

(IX)

durch Reaktion der Verbindungen gemäß Formel VIII mit Aminen der allgemeinen Formel $HNR^4R^5$ mit der oben angegebenen Bedeutung von $R^4$ und $R^5$ in Gegenwart von Quecksilber (II) oxid in einem geeigneten Lösungsmittel, z. B. Äthanol. Das Quecksilberoxid kann auch durch ein organisches Quecksilbersalz, z. B. $Hg(OAc)_2$, ersetzt werden. Verbindungen (B) der allgemeinen Formel I erhält man aus (IX) durch Verseifung mit Hilfe einer wäßrigen Natrium- oder Kaliumhydroxyd-Lösung bei Siedetemperatur.

Die als Ausgangsmaterialien verwendeten Verbindungen der allgemeinen Formel II sind entweder bekannte Verbindungen oder können analog zu bekannten Verbindungen hergestellt werden.

Die Verbindungen der allgemeinen Formel I können entweder als Basen oder in der Form ihrer Salze aus den Reaktionsgemischen isoliert werden.

Sie lassen sich als Basen mit geeigneten anorganischen oder organischen Säuren nach bekannten Verfahren in Salze überführen. Bevorzugt werden physiologisch verträgliche Salze. Hierfür sind als anorganische Säuren beispielsweise Halogenwasserstoffsäuren, z. B. Salzsäure, oder Schwefelsäure und als organische Säuren z. B. Fumarsäure und Maleinsäure geeignet. Zur Herstellung wird die heiße alkoholische Lösung der Base mit der alkoholischen Lösung einer geeigneten Säure versetzt und man erhält nach Ätherzusatz das Salz.

Die folgenden Beispiele dienen zur Erläuterung der Erfindung :

## Beispiel 1

2-(3,4,5,-6-Tetrahydro-2-pyrimidinamino)-6-chlor-1H-2,3-dihydrobenzo[de]isochinolin

Zu einer Suspension von 1,45 g Ammoniumthiocyanat in 50 ml 1,2-Dimethoxyäthan tropft man 2,4 ml Benzoylchlorid, rührt 30 Minuten bei Raumtemperatur und tropft anschließend 4,4 g 2-Amino-6-Chlor-1H-2,3-dihydrobenzo[de]isochinolin in 20 ml 1,2-Dimethoxyäthan zu. Der Niederschlag wird nach einer Stunde abgesaugt, mit Wasser gewaschen und zur Hydrolyse der Benzoylgruppe in 50 ml äthanolischer Natronlauge (2 N) eine Stunde gekocht. Nach Abdampfen des Lösungsmittels wird der Rückstand mit Wasser gewascher und getrocknet.

Der Thioharnstoff wird in 50 ml Äthanol gelöst und mit 1 ml Methyljodid zwei Stunden zum Sieden erhitzt. Nach Abkühlen wird it Äther verdünnt und das S-Methylisothiuronium-Salz abgesaugt. Ausbeute : 3,7 g.

3,7 g Isothiuronium-Verbindung werden in 40 ml n-Amylalkohol mit 0,85 ml 1,3-Diaminopropan bei Siedetemperatur kondensiert. Beim Abkühlen kristallisieren 2,6 g des Hydrojodids des 2-(3,4,5,6-Tetrahydro-2-pyrimidinamino)-6-chlor-1H-2,3-dihydrobenzo[de]isochinolins aus. Nach Umkristallisation aus Essigester/Äthanol erhält man 2,4 g vom Fp. 264-266 °C.

Analog Beispiel 1 werden mit 1,2-Diaminoäthan folgende Imidazoline (Verbindungen A) der allgemeinen Formel I erhalten :

| Beisp. | $R^1$ | $R^2$ | Z | n | Fp. °C | Salz |
|---|---|---|---|---|---|---|
| 2 | H | H | NH | 2 | 258-260 (Z.) | HJ |
| 3 | 6-Cl | H | NH | 2 | 231-232 (Z.) | HCl |
| 4 | $6\text{-}OCH_3$ | H | NH | 2 | 202-205 (Z.) | HCl |
| 5 | $6,7\text{-}(CH_2CH_2)\text{-}$ | | NH | 2 | 285-290 (Z.) | HCl |

($R^3$ und $R^4$ zusammen bedeuten $—(CH_2)_n—$)

Beispiel 6

2-(3,4-Dihydro-2H-pyrrol-5-amino)-1H-2,3-dihydro-benzo[de]isochinolin

1,84 g 2-Amino-1H-2,3-dihydro-benzo[de]isochinolin und 2,0 g 5-Methoxy-3,4-dihydro-2H-pyrrol werden 30 Minuten auf 150 °C erhitzt, das Reaktionsgemisch wird in $CH_2Cl_2$ aufgenommen und mit verdünnter wässriger Salzsäure extrahiert. Der Ruckstand der wäßrigen Phase wird durch Chromatografie an Kieselgel gereinigt und anschließend aus Essigester/Äthanol kristallisiert. Man erhält 2-(3,4-Dihydro-2H-pyrrol-5-amino)-1H-2,3-dihydro-benzo[de]isochinolin als Hydrochlorid, Fp. 242-243 °C.

Beispiel 7

2-(2-Oxazolin-2-yl-amino)-1H-2,3-dihydro-benzo[de]isochinolin

Zu 3,1 g 2-Amino-1H-2,3-dihydro-benzo[de]isochinolin werden in 60 ml Toluol bei 20-30 °C 1,46 ml Chloräthylisocyanat in 10 ml Toluol getropft. Nach einer Stunde wird der Niederschlag abgesaugt, mit Toluol gewaschen und getrocknet. Ausbeute : 4,2 g N-(2-Chloräthyl)-N'-(1H-2,3-dihydro-benzo[de]isochinolin-2-yl)-harnstoff.

Der Ringschluß zum Oxazolin erfolgt durch Erhitzen des Harnstoffs in Wasser bis zur vollständigen Lösungen. Nach Umkristallisation aus Äthanol erhält man 1,5 g 1-(2-Oxazolin-2-yl-amino)-1H-2,3-dihydro-benzo[de]isochinolinhydrochlorid. Fp. 276 °C (Z.).

Beispiel 8

Analog Beispiel 7 wurde folgende Verbindung mit Chloräthylisothiocyanat über den entsprechenden Thioharnstoff hergestellt :

2-(2-Thiazolin-2-yl-amino)-1H-2,3-dihydro-benzo[de]isochinolin. Fp. (Base) 188-190 °C. Fp. (Hydrochlorid) : 236-237 °C.

Beispiel 9

(1H-2,3-Dihydro-benzo[de]isochinolin-2-yl)-guanidin

2,2 g (10 mMol) 2-Amino-1H-2,3-dihydro-benzo[de]isochinolin werden mit 0,5 g (1 mMol) Cyanamid in 10 ml n-Amylalkohol 2 Stunden zum Sieden erhitzt. Nach Abdampfen des Lösungsmittels wird der kristalline Rückstand in t-Butanol digeriert und abgesaugt. Die Base wird in Äthanol/Essigester gelöst und durch Zugabe von äthanolischen HCl in das Hydrochlorid überführt. Fp. 246-248 °C (Z.).

Analog Beispiel 9 wurden die folgenden Verbindungen (B) der allgemeinen Formel I hergestellt :

(Siehe Tabelle Seite 8 f.)

| Beisp. | $R^1$ | $R^2$ | $R^3$ | $R^4$ | $R^5$ | Fp. °C | Salz |
|---|---|---|---|---|---|---|---|
| 10 | H | 6-$OCH_3$ | H | H | H | 203 (Z.) | HCl |
| 11 | H | 6-Cl | H | H | H | 148 (Z.) | HCl + $CH_3COOEt$ |
| 12 | 6,7-(-$CH_2CH_2$-) | | H | H | H | 182-185 | HCl |
| 13 | H | H | H | $CH_3$ | $CH_3$ | 290 (Z.) | HCl |

(Z = N—$R^5$)

Beispiel 14

1,2-Dimethyl-3-(1H-2,3-dihydro-benzo[de]isochinolin-2-yl)-guanidin

4,6 g (25 mMol) 2-Amino-1H-2,3-dihydro-benzo[de]isochinolin werden in 30 ml 1,2-Dimethoxyäthan gelöst, und nach Zugabe von 1,85 g (25 mMol) Methylisothiocyanat wird 15 Stunden bei Raumtemperatur gerührt. Man erhält 5 g 1-Methyl-3-(1H-2,3-dihydro-benzo[de]isochinolin-2-yl)-thioharnstoff als kristallinen Niederschlag. Fp. 251-252 °C (Z.)

4,3 g des obigen Thioharnstoffs werden in einem Zweiphasensystem ($CH_2Cl_2/H_2O$) mit 8 g Quecksilberacetat, 15 ml 2 N NaOH und 30 ml Methylaminlösung (40 %ige wässrige Lösung) 1,5 Stunden kräftig gerührt. Nach Filtration über Cellulosepulver wird die $CH_2Cl_2$-Phase eingedampft, der Rückstand in Essigester gelöst und mit äthanolischer Salzsäure das Hydrochlorid des 1,2-Dimethyl-3-(1H-2,3-dihydro-benzo[de]isochinolin-2-yl)-guanidins gefällt. Nach Umkristallisation aus Äthanol/Äther erhält man 2,7 g. Fp. > 300 °C.

Beispiel 15

Analog Beispiel 14 erhält man die folgende Verbindung :

1,2,2-Trimethyl-3-(1H-2,3-dihydro-benzo[de]isochinolin-2-yl)-guanidin-hydrochlorid. Fp. 193-195 °C (Z.)

Beispiel 16

1-Methyl-2-(1H-2,3-dihydro-benzo[de]isochinolin-2-yl)-guanidin

Zu einer Suspension von 2,5 g (33 mMol) Ammoniumthiocyanat in 50 ml 1,2-Dimethoxyäthan werden zunächst 3,5 ml (30 mMol) Benzoylchlorid in 10 ml Dimethoxyäthan getropft, 30 Minuten bei Raumtemperatur gerührt und anschließend 5,3 g (30 Mol) 2-Amino-1H-2,3-dihydro-benzo[de]isochinolin in 50 ml Dimethoxyäthan hinzugefügt. Nach fünfzehnminütigem Rühren bei Raumtemperatur wird der Niederschlag abgesaugt und mit Aceton, dann mit Wasser gewaschen. Man erhält 9 g 1-Benzoyl-3-(1H-2,3-dihydro-benzo[de]isochinolin-2-yl)-thioharnstoff, der analog Beispiel 15 mit Methylamin in Gegenwart von Quecksilberacetat in 1-Benzoyl-2-methyl-3-(1H-2,3-dihydrobenzo[de]isochinolin-2-yl)-guanidin umgewandelt wird. Zur Entfernung der Benzoyl-Gruppe wird das Produkt 8 Stunden mit 2 N wässriger Natronlauge erhitzt. Anschließend extrahiert man mit Diisopropyläther das Guanidin und dampft das Lösungsmittel ab. Aus athanolischer Salzsäure/Essigester erhalt man das Hydrochlorid der im Titel genannten Verbindung. Fp. 235-236 °C (Z.)

Beispiel 17

Herstellung von Tabletten

Tabletten, die die im folgenden genannten Bestandteile enthalten, lassen sich in bekannter Weise herstellen. Sie können zur Behandlung hypotoner Zustände in einer Dosis von 1 bis 2 Tabletten zweimal täglich verwendet werden.

| | |
|---|---|
| (1H-2,3-Dihydro-benzo[de]-isochinolin-2-yl)-guanidin | 1 mg |
| Lactose | 75 mg |
| Maisstärke | 10 mg |

| | |
|---|---|
| Mikrokristalline Cellulose | 8 mg |
| Polyvinylpyrrolidon | 1 mg |
| Magnesiumstearat | 0,5 mg |
| Aerosil | 0,5 mg |

Beispiel 18

Herstellung von Ampullen

Ampullen, die die im folgenden genannten Bestandteile enthalten, lassen sich in bekannter Weise herstellen. Wirkstoff und Natriumchlorid werden in Wasser gelöst und unter Stickstoff in Glasampullen abgefüllt. Sie können zur Behandlung hypotoner Zustände in einer Dosis von 1 bis 2 Ampullen zweimal täglich verwendet werden.

| | |
|---|---|
| (1H-2,3-Dihydro-6-methoxybenzo[de]isochinolin-2-yl)-guanidin | 1 mg |
| Natriumchlorid | 18 mg |
| dest. Wasser ad | 2,0 ml |

Beispiel 19

Herstellung von Tabletten

Tabletten, die die im folgenden genannten Bestandteile enthalten, lassen sich in bekannter Weise herstellen. Sie können zur Behandlung hypotoner Zustände in einer Dosis von 1 bis 2 Tabletten zweimal täglich verwendet werden.

| | |
|---|---|
| 2-(2-Imidazolin-2-yl-amino)-1H-2,3-dihydro-6-chlorbenzo[de]isochinolin | 10 mg |
| Lactose | 70 mg |
| Maisstärke | 10 mg |
| Mikrokristalline Cellulose | 8 mg |
| Polyvinylpyrrolidon | 1 mg |
| Magnesiumstearat | 0,5 mg |
| hochdisperses Siliziumdioxid | 0,5 mg |

Beispiel 20

Herstellung von Ampullen

Ampullen, die die im folgenden genannten Bestandteile enthalten, lassen sich in bekannter Weise herstellen. Wirkstoff und Natriumchlorid werden in Wasser gelöst und unter Stickstoff in Glasampullen abgefüllt. Sie können zur Behandlung hypotoner Zustände in einer Dosis von 1 bis 2 Ampullen zweimal täglich, verwendet werden.

| | |
|---|---|
| Natriumchlorid | 18 mg |
| dest. Wasser ad | 2,0 ml |
| 2-(2-Imidazolin-2-yl-amino)-1H-2,3-dihydro-6-chlorbenzo[de]isochinolin | 5 mg |

**Ansprüche** (für die Vertragsstaaten : BE, CH, DE, FR, GB, IT, LI, NL, SE)

1. Substituierte Benzo[de]isochinoline der allgemeinen Formel I

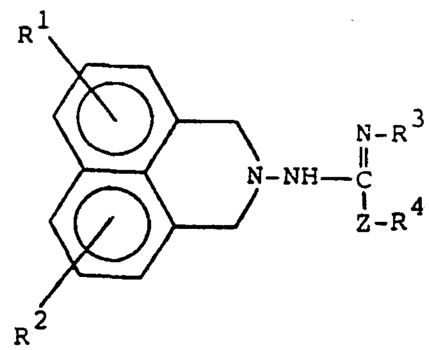

(I)

in der

$R^1$ und $R^2$, die gleich oder verschieden sein können, ein Wasserstoff- oder Halogenatom oder eine Alkoxygruppe mit 1 bis 4 Kohlenstoffatomen oder

$R^1$ und $R^2$ zusammen die Äthylengruppe,

Z Sauerstoff, Schwefel, die Methylengruppe oder die Gruppe N—$R^5$, und entweder

$R^3$ und $R^4$ zusammen die Gruppe —$(CH_2)_n$— bedeuten, worin n die Zahl 2 oder 3 sein kann und wobei $R^5$ Wasserstoff ist (Verbindungen A),

oder, wenn Z die Gruppe N—$R^5$ ist, $R^3$, $R^4$ und $R^5$, die gleich oder verschieden sein können, ein Wasserstoffatom oder eine Alkylgruppe mit 1 bis 4 Kohlenstoffatomen bedeuten (Verbindungen B) sowie deren Säureadditionssalze.

2. Verfahren zur Herstellung von Verbindungen (A) der allgemeinen Formel I gemäß Anspruch 1, worin Z ein Sauerstoff- oder Schwefelatom bedeutet, dadurch gekennzeichnet, daß man Verbindungen der allgemeinen Formel II

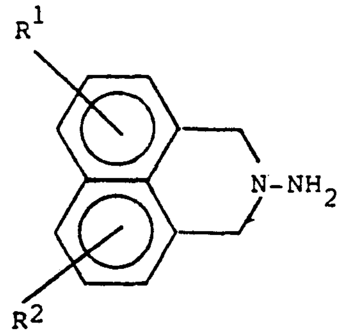

(II)

in der $R^1$ und $R^2$ die oben angegebene Bedeutung haben, mit Halogenalkylisocyanaten oder Halogenalkylisothiocyanaten der allgemeinen Formel III

$$Z=C=N—(CH_2)_n—Y \quad (III)$$

umsetzt, in der Z ein Sauerstoff- oder Schwefelatom ist und n die obengenannte Bedeutung hat und Y ein Chlor-, Brom- oder Jodatom ist, und die zunächst entstehenden Verbindungen der allgemeinen Formel IV

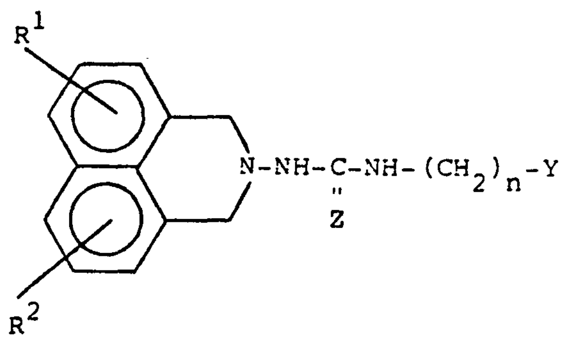

(IV)

in der $R^1$, $R^2$, Z, n und Y die angegebene Bedeutung haben, zu Verbindungen der Formel I cyclisiert.

3. Verfahren zur Herstellung von Verbindungen (A) der allgemeinen Formel I gemäß Anspruch 1, worin Z die Methylengruppe bedeutet, dadurch gekennzeichnet, daß man Verbindungen der allgemeinen Formel II

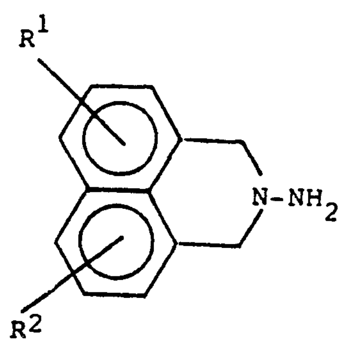

(II)

in der $R^1$ und $R^2$ die oben angegebene Bedeutung haben, mit Iminoäthern der allgemeinen Formel V

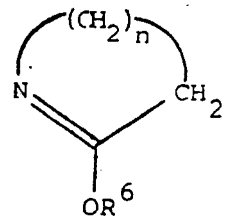

(V)

umsetzt, in der n die vorstehende Bedeutung hat und $R^6$ eine niedere Alkylgruppe ist.

4. Verfahren zur Herstellung von Verbindungen (A) der allgemeinen Formel I gemäß Anspruch 1, worin Z die Iminogruppe bedeutet, dadurch gekennzeichnet, daß man Verbindungen der allgemeinen Formel II

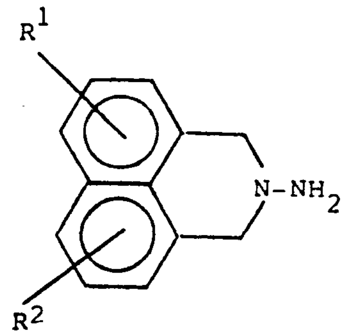

(II)

in der $R^1$ und $R^2$ die oben angegebene Bedeutung haben, mit Benzoylisothiocyanat zu den entsprechenden Benzoylthioharnstoffen und durch Abspalten des Benzoylrests zu den entsprechenden Thioharnstoffen, umsetzt, die durch Alkylierung in die S-Alkylisothiuronium-Salze der allgemeinen Formel VI

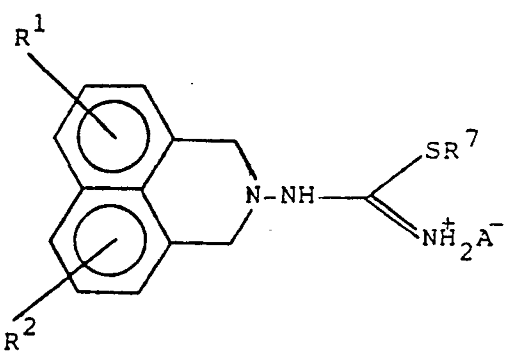

(VI)

überführt werden, in der $R^7$ eine niedere Alkylgruppe und A ein Anion bedeutet sowie $R^1$ und $R^2$ die vorstehend angegebene Bedeutung haben, und diese Verbindungen mit 1,2-Diaminoäthan oder 1,3-Diaminopropan umsetzt.

5. Verfahren zur Herstellung der Verbindungen (B) gemäß Anspruch 1, dadurch gekennzeichnet, daß man Verbindungen der allgemeinen Formel II

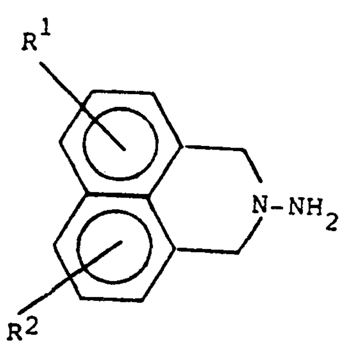

(II)

in der $R^1$ und $R^2$ die oben angegebene Bedeutung haben, mit Cyanamiden umsetzt.

6. Verfahren zur Herstellung der Verbindungen (B) gemäß Anspruch 1, dadurch gekennzeichnet, daß man Verbindungen der allgemeinen Formel II

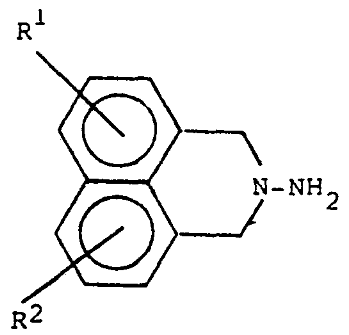

(II)

in der $R^1$ und $R^2$ die oben angegebene Bedeutung haben, mit aliphatischen Isothiocyanaten zu Thiosemicarbaziden der allgemeinen Formel VII

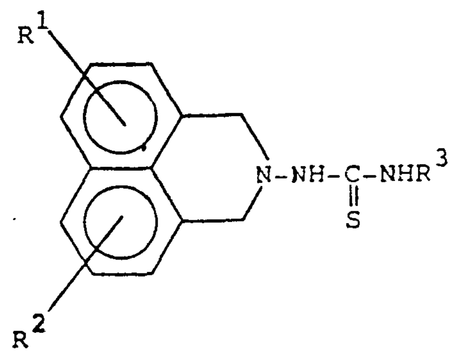

(VII)

umsetzt, in der $R^1$, $R^2$ und $R^3$ die oben angegebene Bedeutung haben, diese Thiosemicarbazide einer S-Methylierung mit Methyljodid unterwirft und die erhaltenen Reaktionsprodukte anschließend mit Ammoniak, primären oder sekundären Aminen umsetzt.

7. Verfahren zur Herstellung der Verbindungen (B) gemäß Anspruch 1, dadurch gekennzeichnet, daß man Verbindungen der allgemeinen Formel II

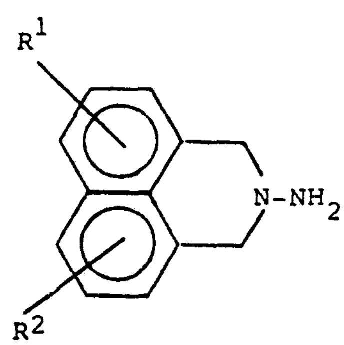

(II)

in der $R^1$ und $R^2$ die oben angegebene Bedeutung haben, mit Benzoylisothiocyanat zu Thiosemicarbaziden der allgemeinen Formel VIII

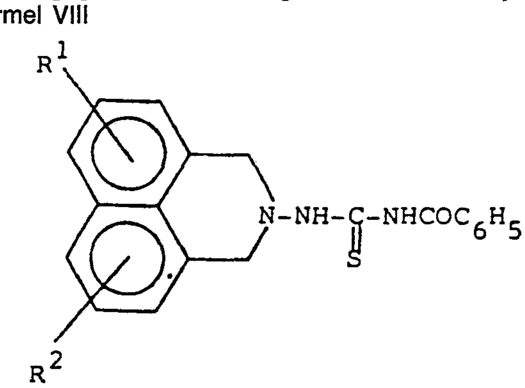

(VIII)

mit der angegebenen Bedeutung für $R^1$ und $R^2$ umsetzt, die durch Reaktion mit Aminen der allgemeinen

Formel $HNR^4R^5$ mit der oben angegebenen Bedeutung von $R^4$ und $R^5$ in die Verbindungen der allgemeinen Formel IX

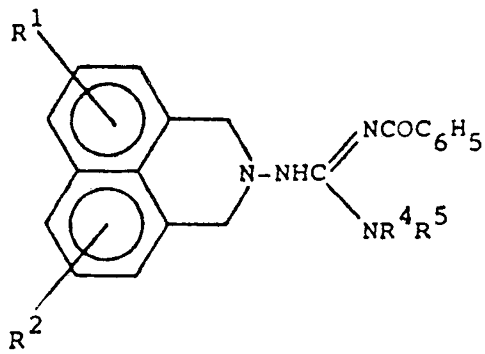

(IX)

mit der angegebenen Bedeutung von $R^1$, $R^2$, $R^4$ und $R^5$ überführt werden, und anschließend die Benzoylgruppe abspaltet.

8. Pharmazeutisches Präparat, dadurch gekennzeichnet, daß es eine oder mehrere der Verbindungen gemäß Anspruch 1 oder deren physiologisch verträgliche Salze und gegebenenfalls übliche Trägerstoffe und/oder Verdünnungsmittel enthält.

9. Verbindungen der allgemeinen Formel I oder deren physiologisch verträgliche Säureadditionssalze gemäß Anspruch 1 zur Anwendung bei hypotonen Zuständen.

10. 2-(2-Imidazolin-2-yl-amino)-1H-2,3-dihydro-benzo[de]isochinolin

11. 2-(2-Imidazolin-2-yl-amino)-1H-2,3-dihydro-6-chlorbenzo[de]isochinolin

12. (1H-2,3-Dihydro-benzo[de]isochinolin-2-yl)-guanidin

13. (1H-2,3,6,7-Tetrahydro-indeno[1,6,7-def]isochinolin-2-yl)-guanidin

**Anspruch** (für den Vertragsstaat AT)

Verfahren zur Herstellung von neuen substituierten Benzo[de]isochinolinen der allgemeinen Formel I

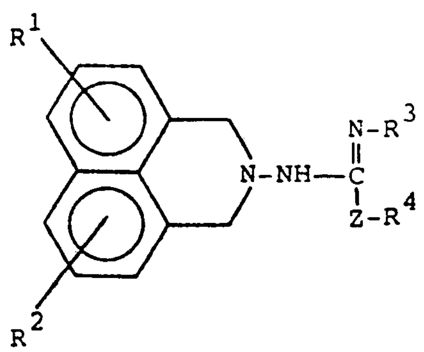

(I)

in der

$R^1$ und $R^2$, die gleich oder verschieden sein können, ein Wasserstoff- oder Halogenatom oder eine Alkoxygruppe mit 1 bis 4 Kohlenstoffatomen oder

$R^1$ und $R^2$ zusammen die Äthylengruppe,

Z Sauerstoff, Schwefel, die Methylengruppe oder die Gruppe N—$R^5$, und entweder

$R^3$ und $R^4$ zusammen die Gruppe $-(CH_2)_n-$ bedeuten, worin n die Zahl 2 oder 3 sein kann und wobei $R^5$ Wasserstoff ist (Verbindungen A),

oder, wenn Z die Gruppe N—$R^5$ ist, $R^3$, $R^4$ und $R^5$, die gleich oder verschieden sein können, ein Wasserstoffatom oder eine Alkylgruppe mit 1 bis 4 Kohlenstoffatomen bedeuten (Verbindungen B)

sowie deren Säureadditionssalzen, dadurch gekennzeichnet, daß man zur Herstellung von Verbindungen (A) der allgemeinen Formel I worin Z ein Sauerstoff- oder Schwefelatom bedeutet, Verbindungen der allgemeinen Formel II

(Siehe Formel II Seite 14 f.)

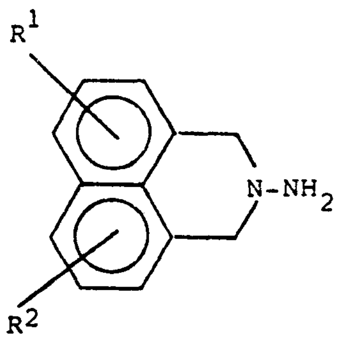

(II)

in der $R^1$ und $R^2$ die oben angegebene Bedeutung haben, mit Halogenalkylisocyanaten oder Halogenalkylisothiocyanaten der allgemeinen Formel III

$$Z = C{=}N{-}(CH_2)_n{-}Y \qquad (III)$$

umsetzt, in der Z ein Sauerstoff- oder Schwefelatom ist und n die obengenannte Bedeutung hat und Y ein Chlor-, Brom- oder Jodatom ist, und die zunächst entstehenden Verbindungen der allgemeinen Formel IV

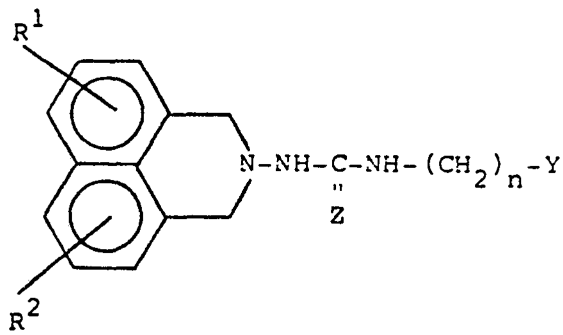

(IV)

in der $R^1$, $R^2$, Z, n und Y die angegebene Bedeutung haben, zu Verbindungen der Formel I cyclisiert und gewünschtenfalls ein Säureadditionssalz daraus herstellt und zur Herstellung von Verbindungen (A) der allgemeinen Formel I, worin Z die Methylengruppe bedeutet, Verbindungen der allgemeinen Formel II

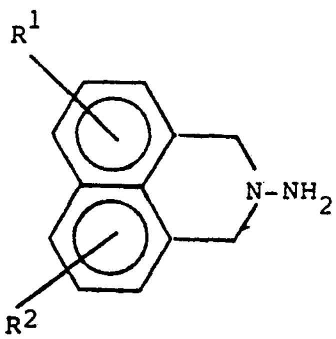

(II)

in der $R^1$ und $R^2$ die oben angegebene Bedeutung haben, mit Iminoäthern der allgemeinen Formel V

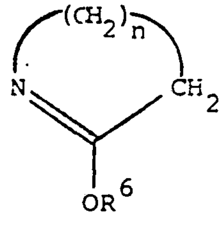

(V)

umsetzt, in der n die vorstehende Bedeutung hat und $R^6$ eine niedere Alkylgruppe ist und aus den erhaltenen Verbindungen der Formel I gewünschtenfalls ein Säureadditionssalz herstellt und zur

Herstellung von Verbindungen (A) der allgemeinen Formel I, worin Z die Iminogruppe bedeutet, Verbindungen der allgemeinen Formel II

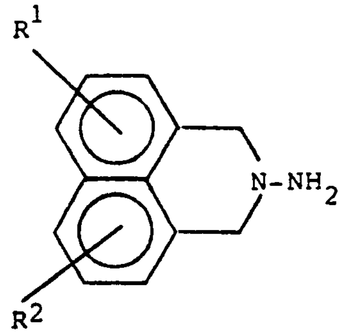

(II)

in der $R^1$ und $R^2$ die oben angegebene Bedeutung haben, mit Benzoylisothiocyanat zu den entsprechenden Benzoylthioharnstoffen und durch Abspalten des Benzoylrests zu den entsprechenden Thioharnstoffen umsetzt, die durch Alkylierung in die S-Alkylisothiuronium-Salze der allgemeinen Formel VI

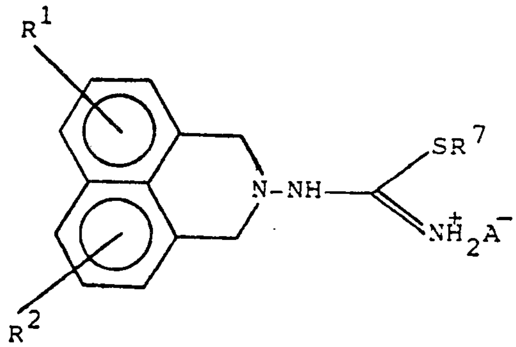

(VI)

überführt werden, in der $R^7$ eine niedere Alkylgruppe und A ein Anion bedeutet sowie $R^1$ und $R^2$ die vorstehend angegebene Bedeutung haben, und diese Verbindungen mit 1,2-Diaminoäthan oder 1,3-Diaminopropan umsetzt und aus den erhaltenen Verbindungen der Formel I gewünschtenfalls ein Säureadditionssalz herstellt und zur Herstellung der Verbindungen (B), Verbindungen der allgemeinen Formel II

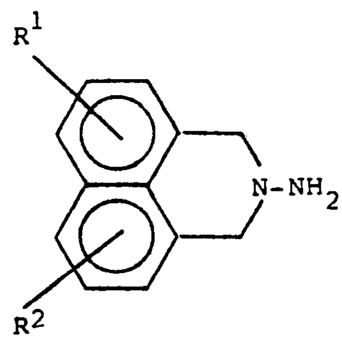

(II)

in der $R^1$ und $R^2$ die oben angegebene Bedeutung haben, mit Cyanamiden umsetzt und aus den erhaltenen Verbindungen der Formel I gewünschtenfalls ein Säureadditionssalz herstellt und zur Herstellung der Verbindungen (B), Verbindungen der allgemeinen Formel II

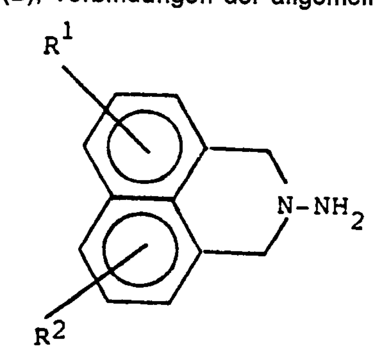

(II)

in der $R^1$ und $R^2$ die oben angegebene Bedeutung haben, mit aliphatischen Isothiocyanaten zu Thiosemicarbaziden der allgemeinen Formel VII

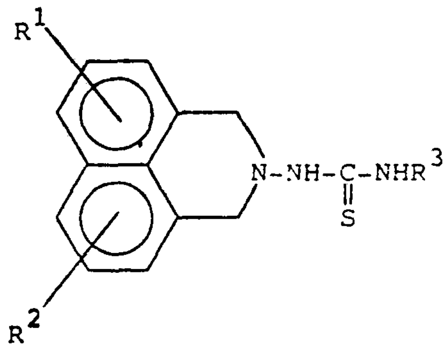

(VII)

umsetzt, in der $R^1$, $R^2$ und $R^3$ die oben angegebene Bedeutung haben, diese Thiosemicarbazide einer S-Methylierung mit Methyljodid unterwirft und die erhaltenen Reaktionsprodukte anschließend mit Ammoniak, primären oder sekundären Aminen umsetzt und aus den erhaltenen Verbindungen der Formel I
gewünschtenfalls ein Säureadditionssalz herstellt und zur Herstellung der Verbindungen (B), Verbindungen der allgemeinen Formel II

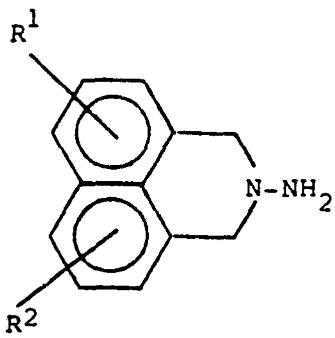

(II)

in der $R^1$ und $R^2$ die oben angegebene Bedeutung haben, mit Benzoylisothiocyanat zu Thiosemicarbaziden der allgemeinen Formel VIII

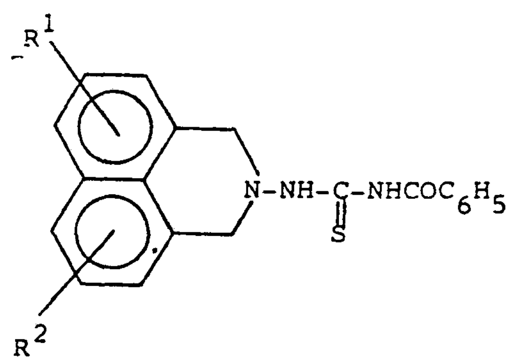

(VIII)

mit der angegebenen Bedeutung für $R^1$ und $R^2$ umsetzt, die durch Reaktion mit Aminen der allgemeinen Formel $HNR^4R^5$ mit der oben angegebenen Bedeutung von $R^4$ und $R^5$ in die Verbindungen der allgemeinen Formel IX

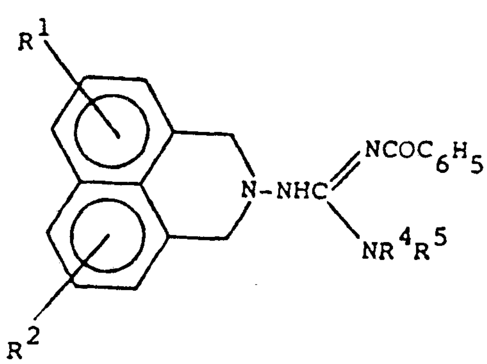

(IX)

mit der angegebenen Bedeutung von $R^1$, $R^2$, $R^4$ und $R^5$ überführt werden, und anschließend die Benzoylgruppe abspaltet und aus den erhaltenen Verbindungen der Formel I gewünschtenfalls ein Säureadditionssalz herstellt.

**Claims** (for the Contracting States : BE, CH, DE, FR, GB, IT, LI, NL, SE)

1. Substituted benzo[de]isoquinolines of the general formula I

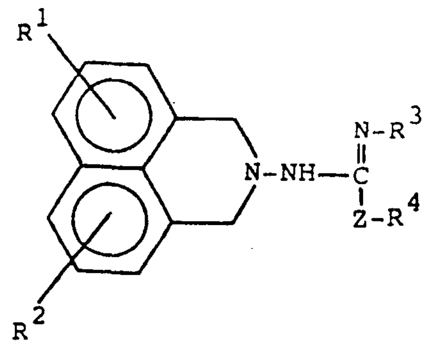

(I)

in which

$R^1$ and $R^2$, which can be identical or different, denote a hydrogen or halogen atom or an alkoxy group having 1 to 4 carbon atoms, or

$R^1$ and $R^2$ together denote the ethylene group,

Z denotes oxygen, sulphur, the methylene group or the group N—$R^5$, and either

$R^3$ and $R^4$ together denote the group $-(CH_2)_n-$ wherein n can be the number 2 or 3, $R^5$ being hydrogen (compounds A),

or, if Z is the group N—$R^5$, $R^3$, $R^4$ and $R^5$, which can be identical or different, denote a hydrogen atom or an alkyl group having 1 to 4 carbon atoms (compounds B),

and also acid addition salts thereof.

2. Process for the preparation of compounds (A) of the general formula I, according to Claim 1, wherein Z denotes an oxygen or sulphur atom, characterised in that compounds of the general formula II

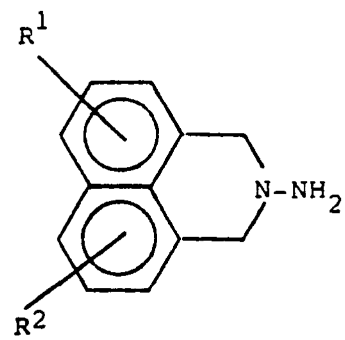

(II)

in which $R^1$ and $R^2$ have the meaning indicated above, are reacted with halogenoalkyl isocyanates or halogenoalkyl isothiocyanates of the general formula III

$$Z = C{=}N{-}(CH_2)_n{-}Y \qquad (III)$$

in which Z is an oxygen or sulphur atom and n has the abovementioned meaning and Y is a chlorine, bromine or iodine atom, and the initially formed compounds of the general formula IV

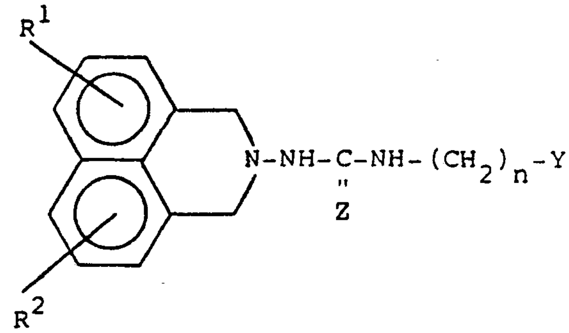

(IV)

in which $R^1$, $R^2$, Z, n and Y have the meaning indicated, are cyclised to give compounds of the formula I.

3. Process for the preparation of compounds (A) of the general formula I, according to Claim 1, wherein Z denotes the methylene group, characterised in that compounds of the general formula II

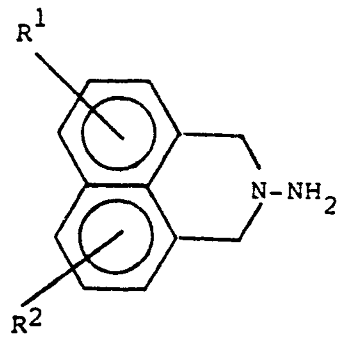

(II)

in which $R^1$ and $R^2$ have the meaning indicated above, are reacted with iminoethers of the general formula V

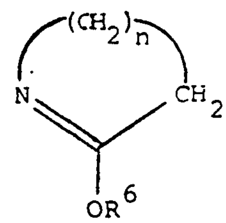

(V)

in which n has the above meaning and $R^6$ is a lower alkyl group.

4. Process for the preparation of compounds (A) of the general formula I, according to Claim 1, wherein Z denotes the imino group, characterised in that compounds of the general formula II

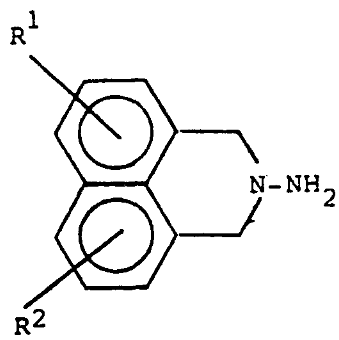

(II)

in which $R^1$ and $R^2$ have the meaning indicated above, are reacted with benzoyl isothiocyanate to give the corresponding benzoyl thioureas and the latter are converted, by elimination of the benzoyl radical, into the corresponding thioureas, which are converted by alkylation into the S-alkylisothiuronium salts of the general formula VI

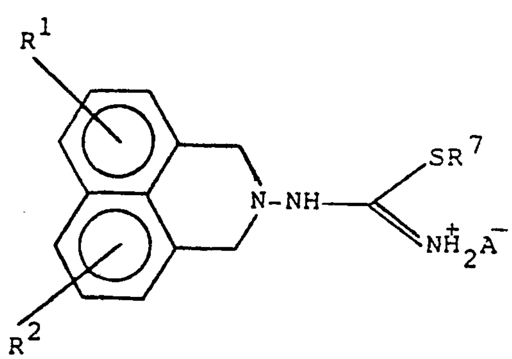

(VI)

in which $R^7$ denotes a lower alkyl group and A denotes an anion, and $R^1$ and $R^2$ have the meaning indicated above, and these compounds are reacted with 1,2-diaminoethane or 1,3-diaminopropane.

5. Process for the preparation of compounds (B) according to Claim 1, characterised in that compounds of the general formula II

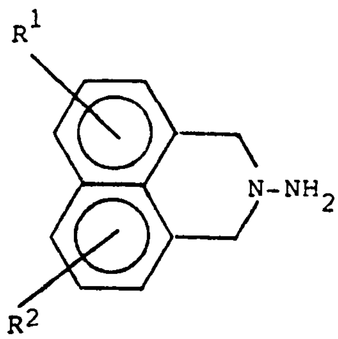

(II)

in which $R^1$ and $R^2$ have the meaning indicated above, are reacted with cyanamides.

6. Process for the preparation of compounds (B) according to Claim 1, characterised in that compounds of the general formula II

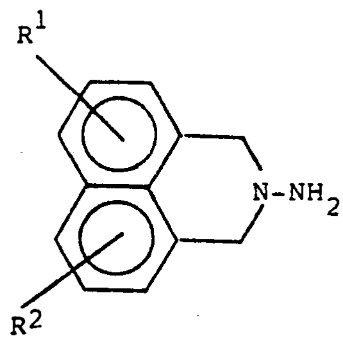

(II)

in which $R^1$ and $R^2$ have the meaning indicated above, are reacted with aliphatic isothiocyanates to give thiosemicarbazides of the general formula VII

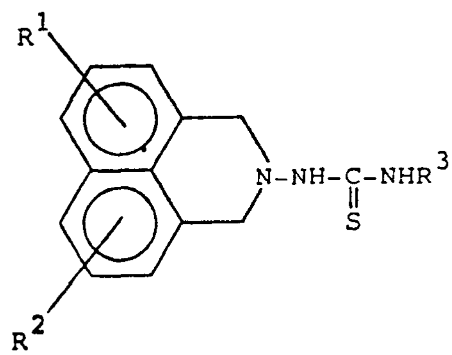

(VII)

in which $R^1$, $R^2$ and $R^3$ have the meaning indicated above, these thiosemicarbazides are subjected to an S-methylation with methyl iodide, and the resulting reaction products are then reacted with ammonia or primary or secondary amines.

7. Process for the preparation of compounds (B) according to Claim 1, characterised in that compounds of the general formula II

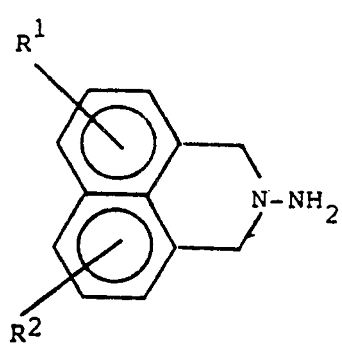

(II)

in which $R^1$ and $R^2$ have the meaning indicated above, are reacted with benzoyl isothiocyanate to give thiosemicarbazides of the general formula VIII

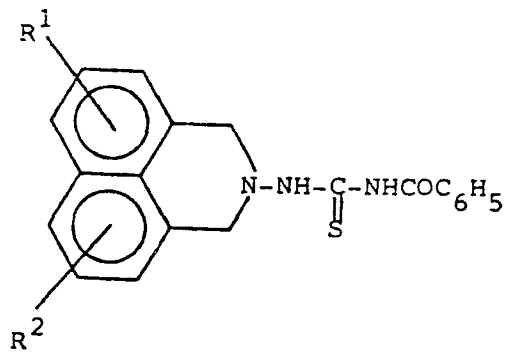

(VIII)

in which $R^1$ and $R^2$ have the meaning indicated, which are converted, by reaction with amines of the general formula $HNR^4R^5$ in which $R^4$ and $R^5$ have the meaning indicated above, into the compounds of the general formula IX

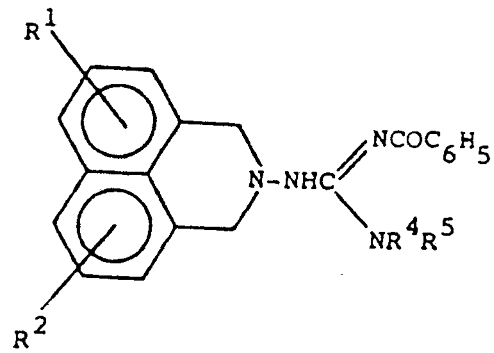

(IX)

in which $R^1$, $R^2$, $R^4$ and $R^5$ have the meaning indicated, and the benzoyl group is then split off.

8. Pharmaceutical formulation characterised in that it contains one or more of the compounds according to Claim 1 or physiologically acceptable salts thereof and, if appropriate, customary excipients and/or diluents.

9. Compounds of the general formula I or physiologically acceptable acid addition salts thereof according to Claim 1 for use in hypotensive states.

10. 2-(2-Imidazolin-2-ylamino)-1H-2,3-dihydrobenzo[de]isoquinoline.

11. 2-(2-Imidazolin-2-ylamino)-1H-2,3-dihydro-6-chlorobenzo[de]isoquinoline.

12. (1H-2,3-Dihydro-benzo[de]isoquinolin-2-yl)-guanidine.

13. (1H-2,3,6,7-Tetrahydro-indeno[1,6,7-def]isoquinolin-2-yl)-guanidine.

**Claim** (for the Contracting State AT)

Process for the preparation of new substituted benzo[de]isoquinolines of the general formula I

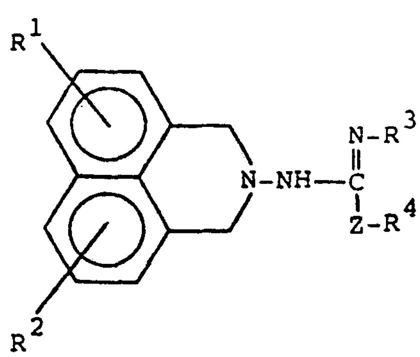

(I)

in which

$R^1$ and $R^2$, which can be identical or different, denote a hydrogen or halogen atom or an alkoxy group having 1 to 4 carbon atoms, or

$R^1$ and $R^2$ together denote the ethylene group,

Z denotes oxygen, sulphur, the methylene group or the group $N—R^5$, and either

$R^3$ and $R^4$ together denote the group $—(CH_2)_n—$ wherein n can be the number 2 or 3 and $R^5$ being hydrogen (compounds A),

or, if Z is the group N—$R^5$, $R^3$, $R^4$ and $R^5$, which can be identical or different, denote a hydrogen atom or an alkyl group having 1 to 4 carbon atoms (compounds B),
and also acid addition salts thereof, characterised in that, in order to prepare compounds (A) of the general formula I wherein Z denotes an oxygen or sulphur atom, compounds of the general formula II

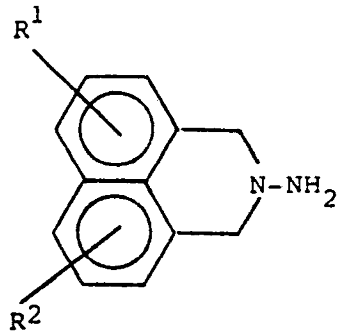

(II)

in which $R^1$ and $R^2$ have the meaning indicated above, are reacted with halogenoalkyl isocyanates or halogenoalkyl isothiocyanates of the general formula III

$$Z=C=N—(CH_2)_n—Y \qquad (III)$$

in which Z is an oxygen or sulphur atom and n has the abovementioned meaning and Y is a chlorine, bromine or iodine atom, and the initially formed compounds of the general formula IV

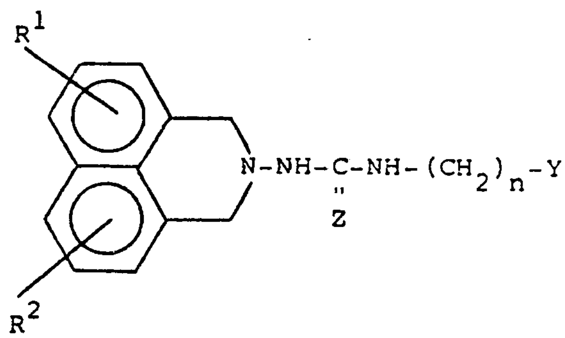

(IV)

in which $R^1$, $R^2$, Z, n and Y have the meaning indicated, are cyclised to give compounds of the formula I and, if desired, an acid addition salt is prepared therefrom, and, in order to prepare compounds (A) of the general formula I wherein Z denotes the methylene group, compounds of the general formula II

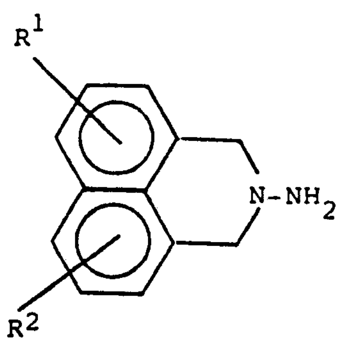

(II)

in which $R^1$ and $R^2$ have the meaning indicated above, are reacted with iminoethers of the general formula V

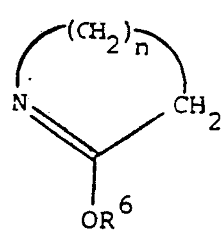

(V)

in which n has the above meaning and $R^6$ is a lower alkyl group, and, if desired, an acid addition salt is prepared from the resulting compounds of the formula I, and, in order to prepare compounds (A) of the general formula I wherein Z denotes the imino group, compounds of the general formula II

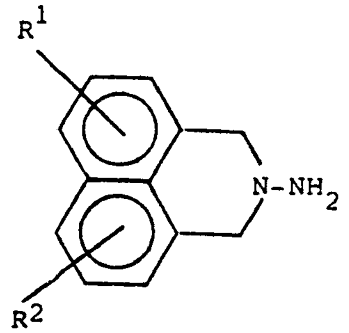

(II)

in which $R^1$ and $R^2$ have the meaning indicated above, are reacted with benzoyl isothiocyanate to give the corresponding benzoyl thioureas, and the latter are converted, by elimination of the benzoyl radical, into the corresponding thioureas, which are converted by alkylation into the S-alkylisothiuronium salts of the general formula VI

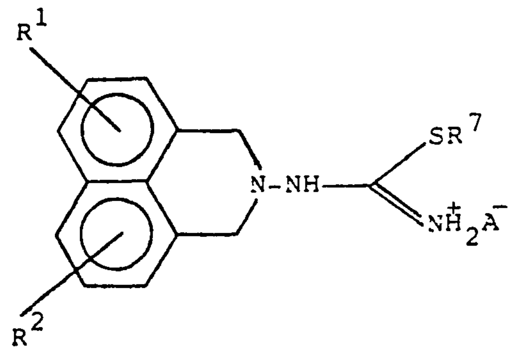

(VI)

in which $R^7$ denotes a lower alkyl group and A denotes an anion, and $R^1$ and $R^2$ have the meaning indicated above, and these compounds are reacted with 1,2-diaminoethane or 1,3-diaminopropane, and, if desired, an acid addition salt is prepared from the resulting compounds of the formula I, and, in order to prepared compounds (B), compounds of the general formula II

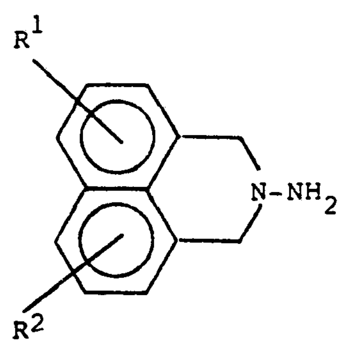

(II)

in which $R^1$ and $R^2$ have the meaning indicated above, are reacted with cyanamides, and, if desired, an acid addition salt is prepared from the resulting compounds of the formula I, and, in order to prepare compounds (B), compounds of the general formula II

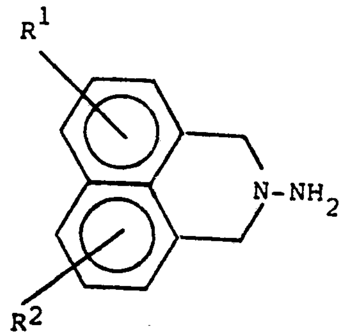

(II)

in which $R^1$ and $R^2$ have the meaning indicated above, are reacted with aliphatic isothiocyanates to give thiosemicarbazides of the general formula VII

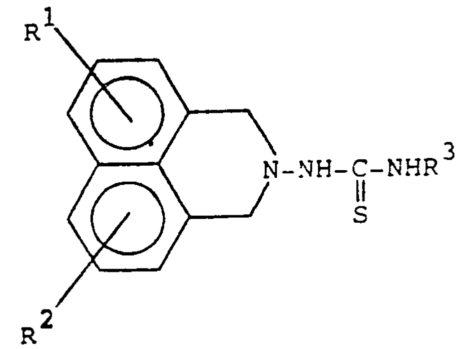

(VII)

in which $R^1$, $R^2$ and $R^3$ have the meaning indicated above, these thiosemicarbazides are subjected to an S-methylation with methyl iodide, and the resulting reaction products are then reacted with ammonia or primary or secondary amines, and, if desired, an acid addition salt is prepared from the resulting compounds of the formula I, and, in order to prepare compounds (B), compounds of the general formula II

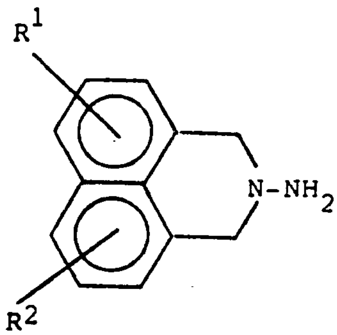

(II)

in which $R^1$ and $R^2$ have the meaning indicated above, are reacted with benzoyl isothiocyanate to give thiosemicarbazides of the general formula VIII

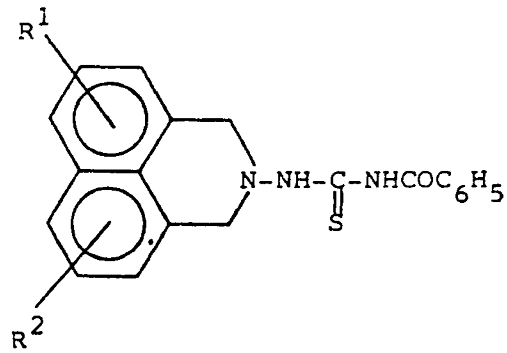

(VIII)

in which $R^1$ and $R^2$ have the meaning indicated, which are converted, by reaction with amines of the general formula $HNR^4R^5$ in which $R^4$ and $R^5$ have the meaning indicated above, into the compounds of the general formula IX

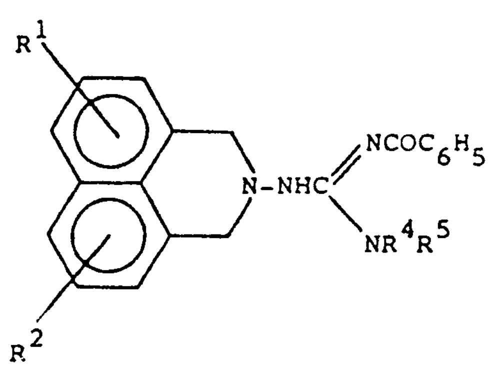

(IX)

in which $R^1$, $R^2$, $R^4$ and $R^5$ have the meaning indicated, and the benzoyl group is then split off, and if desired, an acid addition salt is prepared from the resulting compounds of the formula I.

**Revendications** (pour les Etats contractants : BE, CH, DE, FR, GB, IT, LI, NL, SE)

1. Benzo[de]isoquinoléines substituées de formule générale I

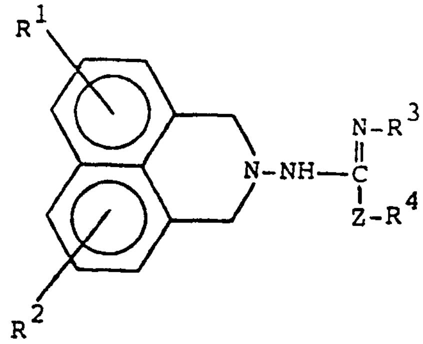

(I)

dans laquelle

$R^1$ et $R^2$, qui peuvent être identiques ou différents, représentent chacun un atome d'hydrogène, un atome d'halogène ou un groupe alcoxy contenant 1 à 4 atomes de carbone, ou

$R^1$ et $R^2$ ensemble représentent le groupe éthylène,

Z représente l'oxygène, le soufre, le groupe méthylène ou le groupe N—$R^5$ et, soit

$R^3$ et $R^4$ ensemble représentent le groupe $—(CH_2)_n$ où n peut être le nombre 2 ou 3, $R^5$ représentant l'hydrogène (composés A),

soit, lorsque Z représente le groupe N—$R^5$, $R^3$, $R^4$ et $R^5$, qui peuvent être identiques ou différents, représentent chacun un atome d'hydrogène ou un groupe alkyle contenant 1 à 4 atomes de carbone (composés B),

ainsi que leurs sels d'addition d'acide.

2. Procédé de préparation de composés (A) de formule générale I suivant la revendication 1 dans laquelle Z représente un atome d'oxygène ou un atome de soufre, caractérisé en ce qu'on fait réagir des composés de formule générale II

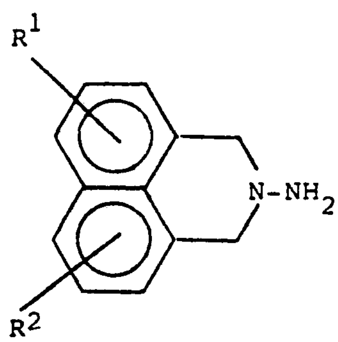

(II)

dans laquelle $R^1$ et $R^2$ ont les significations indiquées ci-dessus, avec des halogénalkylisocyanates ou des halogénalkylisothiocyanates de formule générale III

$$Z = C{=}N{—}(CH_2)_n{—}Y \qquad (III)$$

dans laquelle Z représente un atome d'oxygène ou un atome de soufre, n a la signification indiquée ci-dessus et Y représente un atome de chlore, un atome de brome ou un atome d'iode, et on cyclise les composés tout d'abord formés de formule générale IV

(Voir formule IV page 25)

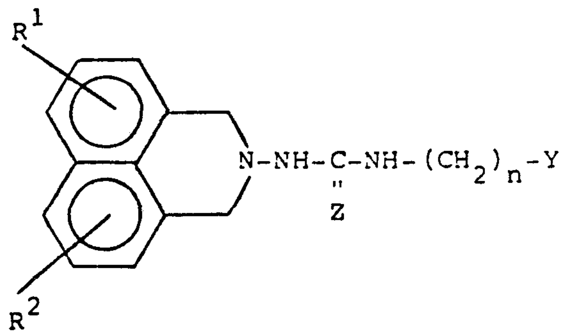

(IV)

dans laquelle $R^1$, $R^2$, Z, n et Y ont les significations indiquées, en composés de formule I.

3. Procédé de préparation de composés (A) de formule générale I suivant la revendication 1 dans laquelle Z représente le groupe méthylène, caractérisé en ce qu'on fait réagir des composés de formule générale II

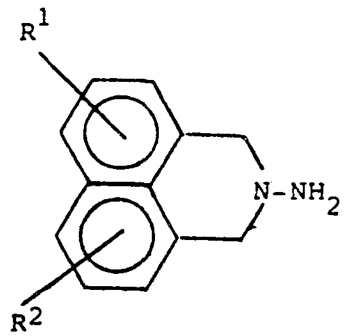

(II)

dans laquelle $R^1$ et $R^2$ ont les significations indiquées ci-dessus, avec des imino-éthers de formule générale V

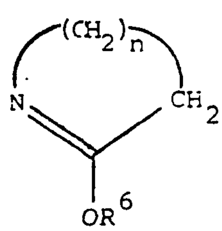

(V)

dans laquelle n a la signification indiquée ci-dessus et $R^6$ représente un groupe alkyle inférieur.

4. Procédé de préparation de composés (A) de formule générale I suivant la revendication 1 dans laquelle Z représente le groupe imino, caractérisé en ce qu'on fait réagir des composés de formule générale II

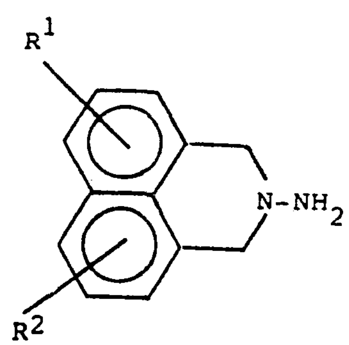

(II)

dans laquelle $R^1$ et $R^2$ ont les significations indiquées ci-dessus, avec du benzoylisothiocyanate, pour obtenir les benzoylthiourées correspondantes et, par séparation du radical benzoyle, pour obtenir les thiourées correspondantes que l'on transforme, par alkylation, en sels de S-alkylisothiuronium de formule générale VI

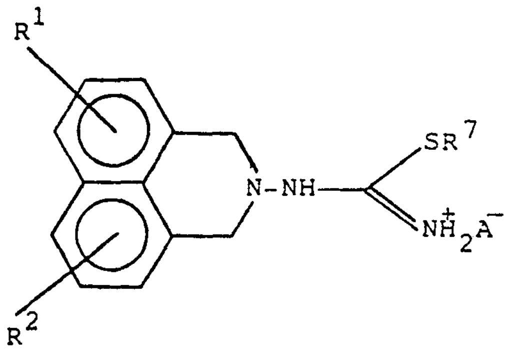

(VI)

dans laquelle $R^7$ représente un groupe alkyle inférieur et A représente un anion, tandis que $R^1$ et $R^2$ ont les significations indiquées ci-dessus, puis on fait réagir ces composés avec le 1,2-diamino-éthane ou le 1,3-diaminopropane.

5. Procédé de préparation des composés (B) suivant la revendication 1, caractérisé en ce qu'on fait réagir des composés de formule générale II

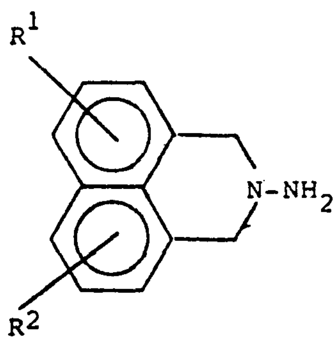

(II)

dans laquelle $R^1$ et $R^2$ ont les significations indiquées ci-dessus, avec des cyanamides.

6. Procédé de préparation des composés (B) suivant la revendication 1, caractérisé en ce qu'on fait réagir des composés de formule générale II

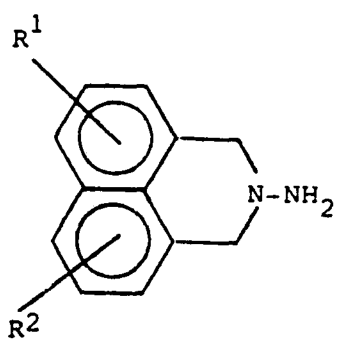

(II)

dans laquelle $R^1$ et $R^2$ ont les significations indiquées ci-dessus, avec des isothiocyanates aliphatiques pour obtenir des thiosemicarbazides de formule générale VII

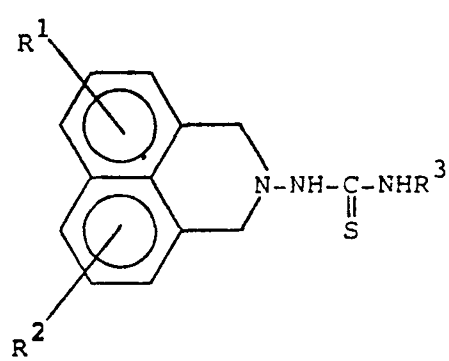

(VII)

dans laquelle $R^1$, $R^2$ et $R^3$ ont les significations indiquées ci-dessus, on soumet ces thiosemicarbazides à une S-méthylation avec de l'iodure de méthyle, puis on fait réagir les produits réactionnels obtenus avec l'ammoniac, des amines primaires ou des amines secondaires.

7. Procédé de préparation des composés (B) suivant la revendication 1, caractérisé en ce qu'on fait réagir des composés de formule générale II

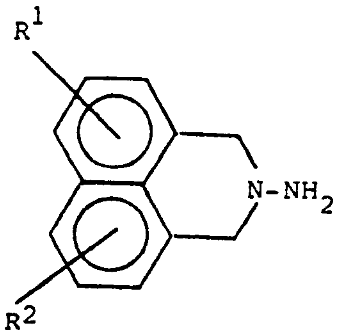

(II)

dans laquelle $R^1$ et $R^2$ ont les significations indiquées ci-dessus, avec du benzoylisothiocyanate pour obtenir des thiosemicarbazides de formule générale VIII

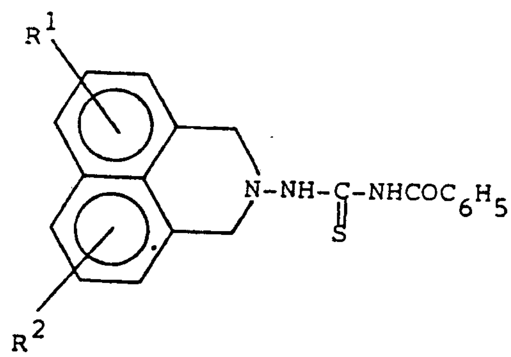

(VIII)

dans laquelle $R^1$ et $R^2$ ont les significations indiquées puis, par réaction avec des amines de formule générale $HNR^4R^5$ dans laquelle $R^4$ et $R^5$ ont les significations indiquées ci-dessus, on transforme ces thiosemicarbazides en composés de formule générale IX

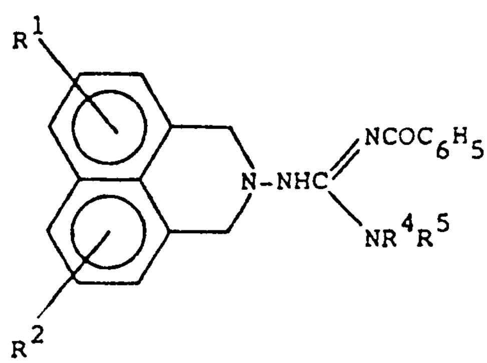

(IX)

dans laquelle $R^1$, $R^2$, $R^4$ et $R^5$ ont les significations indiquées et ensuite, on sépare le groupe benzoyle.

8. Préparation pharmaceutique caractérisée en ce qu'elle contient un ou plusieurs des composés suivant la revendication 1 ou leurs sels physiologiquement compatibles et éventuellement des substances supports et/ou des diluants habituels.

9. Composés de formule générale I ou leurs sels d'addition d'acide physiologiquement compatibles suivant la revendication 1, en vue de les utiliser dans le cas d'états hypotoniques.

10. La 2-(2-imidazolin-2-yl-amino)-1H-2,3-dihydro-benzo[de]isoquinoléine.

11. La 2-(2-imidazolin-2-yl-amino)-1H-2,3-dihydro-6-chloro-benzo[de]isoquinoléine.

12. La (1H-2,3-dihydro-benzo[de]isoquinoléine-2-yl)-guanidine.

13. La (1H-2,3,6,7-tétrahydro-indéno[1,6,7-def]isoquinoléine-2-yl)-guanidine.

**Revendication** (pour l'Etat contractant AT)

Procédé de préparation de nouvelles benzo[de]isoquinoléines substituées de formule générale I

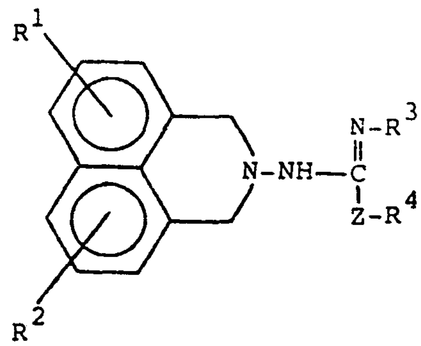

(I)

dans laquelle

$R^1$ et $R^2$, qui peuvent être identiques ou différents, représentent chacun un atome d'hydrogène, un atome d'halogène ou un groupe alcoxy contenant 1 à 4 atomes de carbone, ou

$R^1$ et $R^2$ ensemble représentent le groupe éthylène,

Z représente l'oxygène, le soufre, le groupe méthylène ou le groupe N—$R^5$ et soit

$R^3$ et $R^4$ ensemble représentent le groupe $—(CH_2)_n—$ où n peut être le nombre 2 ou 3, $R^5$ étant l'hydrogène (composés A),

soit, lorsque Z représente le groupe N—$R^5$, $R^3$, $R^4$ et $R^5$, qui peuvent être identiques ou différents, représentent chacun un atome d'hydrogène ou un groupe alkyle contenant 1 à 4 atomes de carbone (composés B),

ainsi que de leurs sels d'addition d'acide, caractérisé en ce que, pour préparer des composés (A) de formule générale I dans laquelle Z représente un atome d'oxygène ou un atome de soufre, on fait réagir des composés de formule générale II

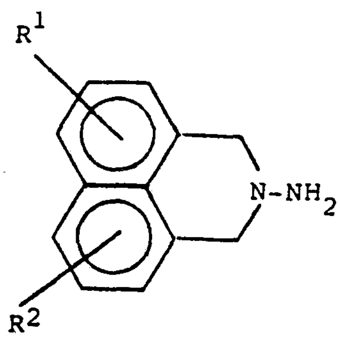

(II)

dans laquelle $R^1$ et $R^2$ ont les significations indiquées ci-dessus, avec des halogénalkylisocyanates ou des halogénalkylisothiocyanates de formule générale III

$$Z = C=N—(CH_2)_n—Y \qquad (III)$$

dans laquelle Z représente un atome d'oxygène ou un atome de soufre, n a la signification indiquée ci-dessus et Y représente un atome de chlore, un atome de brome ou un atome d'iode, puis on cyclise les composés tout d'abord formés de formule générale IV

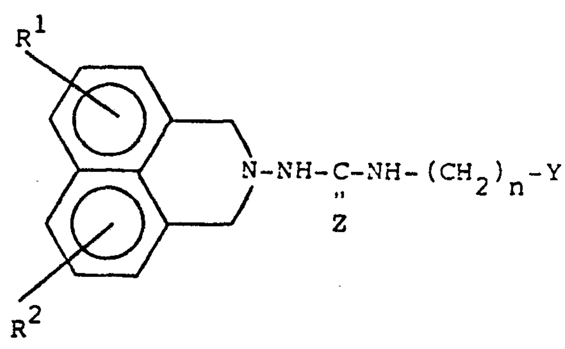

(IV)

dans laquelle $R^1$, $R^2$, Z, n et Y ont les significations indiquées, en composés de formule I et, si on le désire, on prépare un sel d'addition d'acide à partir de ces composés tandis que, pour préparer des composés (A) de formule générale I dans laquelle Z représente le groupe méthylène, on fait réagir des composés de formule générale II

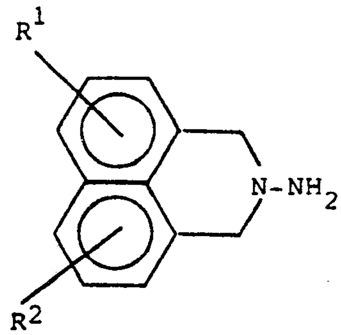

(II)

dans laquelle $R^1$ et $R^2$ ont les significations indiquées ci-dessus, avec des imino-éthers de formule générale V

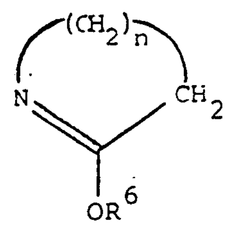

(V)

dans laquelle n a la signification indiquée ci-dessus et $R^6$ représente un groupe alkyle inférieur et, si on le désire, à partir des composés obtenus de formule I, on prépare un sel d'addition d'acide et, pour préparer des composés (A) de formule générale I dans laquelle Z représente le groupe imino, on fait réagir des composés de formule générale II

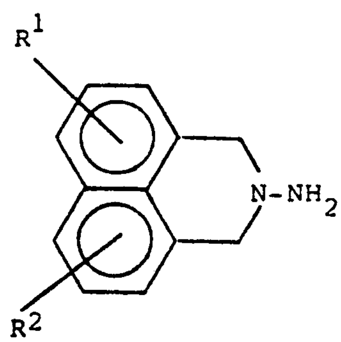

(II)

dans laquelle $R^1$ et $R^2$ ont les significations indiquées ci-dessus, avec du benzoylisothiocyanate pour obtenir les benzoylthiourées correspondantes et, par séparation du radical benzoyle, pour obtenir les thiourées correspondantes que l'on transforme, par alkylation, en sels de S-alkylisothiuronium de formule générale VI

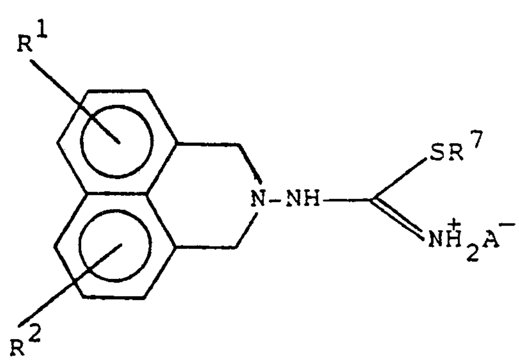

(VI)

dans laquelle $R^7$ représente un groupe alkyle inférieur et A représente un anion, tandis que $R^1$ et $R^2$ ont les significations indiquées ci-dessus, puis on fait réagir ces composés avec le 1,2-diaminoéthane ou le 1,3-diaminopropane et, si on le désire, à partir des composés obtenus de formule I, on prépare un sel d'addition d'acide tandis que, pour préparer des composés (B), on fait réagir des composés de formule générale II

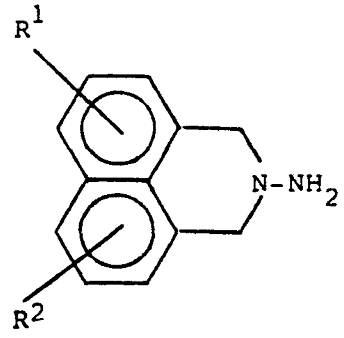

(II)

dans laquelle $R^1$ et $R^2$ ont les significations indiquées ci-dessus, avec des cyanamides et, si on le désire, à partir des composés obtenus de formule I, on prépare un sel d'addition d'acide et, pour préparer des composés (B), on fait réagir des composés de formule générale II

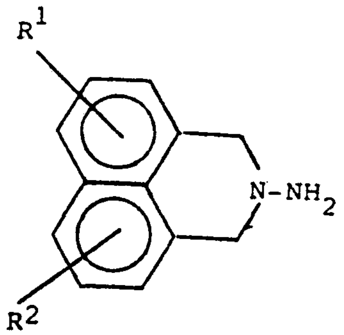

(II)

dans laquelle $R^1$ et $R^2$ ont les significations indiquées ci-dessus, avec des isothiocyanates aliphatiques pour obtenir des thiosemicarbazides de formule générale VII

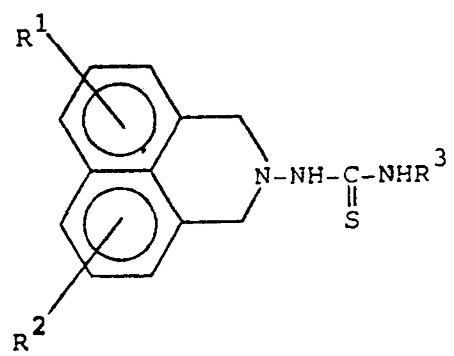

(VII)

dans laquelle $R^1$, $R^2$ et $R^3$ ont les significations indiquées ci-dessus, on soumet ces thiosemicarbazides à une S-méthylation avec l'iodure de méthyle, puis on fait réagir les produits réactionnels obtenus avec l'ammoniac, des amines primaires ou des amines secondaires et, si on le désire, à partir des composés obtenus de formule I, on prépare un sel d'addition d'acide et, pour préparer des composés (B), on fait réagir des composés de formule générale II

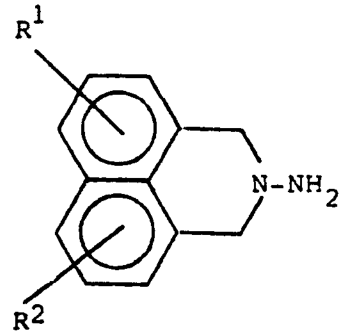

(II)

dans laquelle $R^1$ et $R^2$ ont les significations indiquées ci-dessus, avec du benzoylisothiocyanate pour obtenir des thiosemicarbazides de formule générale VIII

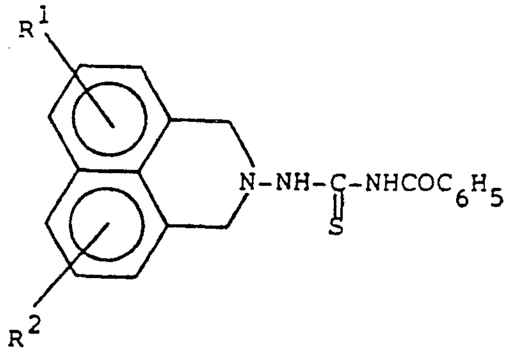

(VIII)

dans laquelle $R^1$ et $R^2$ ont les significations indiquées et, par réaction avec des amines de formule générale $HNR^4R^5$ dans laquelle $R^4$ et $R^5$ ont les significations indiquées ci-dessus, on transforme ces thiosemicarbazides en composés de formule générale IX

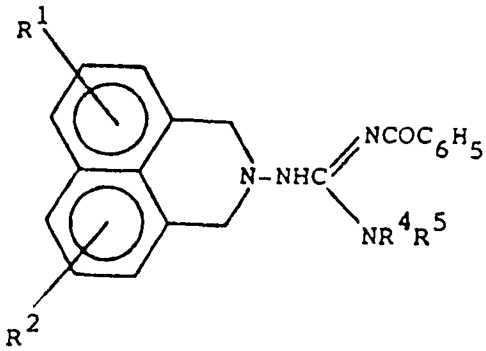

(IX)

dans laquelle $R^1$, $R^2$, $R^4$ et $R^5$ ont les significations indiquées, puis on sépare le groupe benzoyle et, si on le désire, à partir des composés obtenus de formule I, on prépare un sel d'addition d'acide.